Europäisches Patentamt

European Patent Office

Office européen des brevets

(19)

(11) EP 0 983 391 B1

(12) FASCICULE DE BREVET EUROPEEN

(45) Date de publication et mention
de la délivrance du brevet:
**25.08.2004 Bulletin 2004/35**

(51) Int Cl.7: **C12Q 1/68**, C12N 15/10,
C12N 15/85, A01K 67/027

(21) Numéro de dépôt: **98933758.9**

(22) Date de dépôt: **30.06.1998**

(86) Numéro de dépôt international:
**PCT/FR1998/001394**

(87) Numéro de publication internationale:
**WO 1999/001574 (14.01.1999 Gazette 1999/02)**

(54) **PROCEDE DE DIAGNOSTIC DE LA MALADIE D'ALZHEIMER**

DIAGNOSTISCHES VERFAHREN DER ALZHEIMER-ERKRANKUNG

METHOD FOR DIAGNOSING ALZHEIMER DISEASE

(84) Etats contractants désignés:
**BE CH DE DK ES FR GB IT LI LU NL SE**

(30) Priorité: **01.07.1997 FR 9708284**

(43) Date de publication de la demande:
**08.03.2000 Bulletin 2000/10**

(73) Titulaires:
- **INSTITUT PASTEUR DE LILLE**
  **59019 Lille Cédex (FR)**
- **INSTITUT NATIONAL DE LA SANTE ET**
  **DE LA RECHERCHE MEDICALE (INSERM)**
  **75013 Paris (FR)**

(72) Inventeurs:
- **CHARTIER-HARLIN, Marie-Christine**
  **59139 WATIGNIES (FR)**
- **LAMBERT, Jean-Charles**
  **Birmingham B15 2QZ (GB)**
- **AMOUYEL, Philippe**
  **F-59700 Marcq en Baroeul (FR)**

(74) Mandataire: **Jacobson, Claude et al**
**Cabinet Lavoix**
**2, Place d'Estienne d'Orves**
**75441 Paris Cedex 09 (FR)**

(56) Documents cités:
WO-A-95/16791        WO-A-95/24504
WO-A-95/29257        US-A- 5 508 167

- CHARTIER-HARLIN M C ET AL.: "Apoliprotein E, epsilon4 allele as a major risk factor for sporadic early and late-onset forms of Alzheimer's disease: analysis of the 19q13.2 chromosomal region" HUMAN MOLECULAR GENETICS, vol. 3, no. 4, 1994, pages 569-574, XP002059261
- CORDER E H ET AL: "GENE DOSE OF APOLIPOPROTEIN E TYPE 4 ALLELE AND THE RISK OF ALZHEIMER'S DISEASE IN LATE ONSET FAMILIES" SCIENCE, vol. 261, 13 août 1993, pages 921-923, XP000619874 cité dans la demande
- POIRIER J ET AL: "APOLIPOPROTEIN E POLYMORPHISM AND ALZHEIMER'S DISEASE" LANCET THE, vol. 342, 18 septembre 1993, pages 697-699, XP000197782
- XU P-T ET AL.: "Human apolipoprotein E2, E3 and E4 isoform-specific transgenic mice: Human-like pattern of glial and neuronal immunoreactivity in central nervous system not observed in wild-type mice" NEUROBIOLOGY OF DISEASE, vol. 3, 1996, pages 229-245, XP002084385
- JAEN J C ET AL.: "Cholinomimetics and Alzheimer's disease" BIOORGANIC & MEDICINAL CHEMISTRY LETTERS, vol. 2, no. 8, 1992, pages 777-780, XP002084386
- PAIK Y-K ET AL.: "Nucleotide sequence and structure of the human apoliloprotein E gene" PROCEEDINSG OF THE NATIONAL ACADEMY OF SCIENCES USA, vol. 82, 1985, pages 3445-3449, XP002059262 cité dans la demande

EP 0 983 391 B1

- LAMBERT J-C ET AL.: "A new polymorphism in the APOE promotor associated with risk of developing Alzheimer's disease" HUMAN MOLECULAR GENETICS, vol. 7, no. 3, 1998, pages 533-540, XP002084387
- ARTIGA M J ET AL.: "Allelic polymorphisms in the transcriptional regulatory region of apolipoprotein E gene" FEBS LETTERS, vol. 421, 1998, pages 105-108, XP002084388
- BULLIDO M J ET AL.: "A polymorphism in the regulatory region of APOE associated with risk for Alzheimer's dementia." NATURE GENETICS, vol. 18, no. 1, 1998, pages 69-71, XP002059428
- LAMBERT J-C ET AL.: "Distortion of allelic expression of apolipoprotein E in Alzheimer's disease" HUMAN MOLECULAR GENETICS, vol. 6, no. 12, 1997, pages 2151-2154, XP002059263
- LAMBERT J-C ET AL.: "Pronounced impact of Th1/E47cs mutation compared with -491 AT mutation on neural APOE gene expression and risk of developing Alzheimer's disease" HUMAN MOLECULAR GENETICS, vol. 7, no. 9, 1998, pages 1511-1516, XP002084389
- MUI S. ET AL: 'A newly identified polymorphism in the apolipoprotein E enhancer gene region is associated with Alzheimer's disease and strongly with the gamma-4 allele' NEUROLOGY vol. 47, no. 1, Juillet 1996, pages 196 - 201

**Description**

La présente invention concerne un procédé de diagnostic de la maladie d'Alzheimer.

La maladie d'Alzheimer est une démence neurodégénérative caractérisée par une perte de neurones corticaux associée à des plaques β-amyloïdes, des enchevêtrements de neurofibrilles et, dans la plupart des cas, une angiopathie amyloïde. Il existe de fortes présomptions pour une influence génétique dans l'étiologie de la maladie d'Alzheimer (WO 94/01772).

Cette composante génétique a été mise en évidence depuis de nombreuses années par des observations indirectes qui suggèrent une hérédité sur le mode autosomique dominant et une pénétrance dépendante de l'âge pour expliquer certaines formes familiales de la maladie d'Alzheimer. Des études de génétique moléculaire récentes ont permis d'isoler des gènes putatifs de la maladie d'Alzheimer par la recherche de marqueurs génétiques polymorphes spécifiques de chromosomes (Bird et al., 1989, Neurobiology of Aging 10, 432-434).

Quatre localisations chromosomiques ont été décrites comme étant impliquées : trois sur les chromosomes 1, 14 et 21 dans les formes familiales à survenue précoce (âge de survenue inférieur à 60 ans), et une sur le chromosome 19 dans les formes familiales et sporadiques à survenue tardive. Deux études de liaison ont suggéré que la région chromosomique 19q13.2 était associée à des formes de maladie d'Alzheimer familiales à survenue tardive (Pericak-Vance et al, Am. J. Hum. Genet. (1991), 48, 1034-1050). Au sein de cette région chromosomique, le groupe de gènes d'apolipoprotéines (APO) E-CI-CI'-CII est une zone candidate. Parmi les produits de ces gènes, l'apolipoprotéine E (APOE) est particulièrement impliquée dans le système nerveux APOE est présente dans les plaques séniles et possède une affinité de liaison avec le peptide Aβ. L'APOE se caractérise par trois allèles majeurs ε2, ε3, ε4. Strittmatter et al. (Proc. Natl. Acad. Sci. (1993) 90, 177-181) ont décrit une fréquence augmentée de l'allèle ε4 du gène APOE dans les formes familiales de la maladie d'Alzheimer à survenue tardive. Cette observation a été confirmée pour les formes familiales (Corder et al., Science (1993), 261, 921-923) et les formes sporadiques de la maladie d'Alzheimer (Corder et al., Science (1993), 261, 921-923; Saunders et al., Neurology (1993), 13, 1467-1472).

FR-2 716 894 décrit un procédé permettant de pronostiquer pour un malade donné les risques de développer la maladie d'Alzheimer par rapport à la population générale. Ce procédé repose sur la. mise en évidence des allèles ε4 du gène APOE, des allèles courts du marqueur D19S178 et des allèles longs du gène APO CII tous localisés sur le chromosome 19.

Plusieurs hypothèses permettent d'expliquer ce phénomène.

Des travaux récents des inventeurs auteurs de la présente invention viennent à présent confirmer l'hypothèse de l'existence d'au moins une autre mutation fonctionnelle dans la région 19q13.2.

En effet, en plus d'un effet fonctionnel propre du polymorphisme de l'apolipoprotéine E, les études des inventeurs montrent des différences de niveaux d'expression significatifs chez les sujets malades par rapport aux témoins sains, ce qui indique qu'une ou plusieurs mutations dans les régions de régulation du gène APOE sont impliquées dans la survenue de la maladie d'Alzheimer. De plus, des différences d'expression relatives sont retrouvées chez les témoins.

Les inventeurs ont plus particulièrement mis en évidence un nouveau polymorphisme dans la région du promoteur du gène codant pour la protéine apolipoprotéine E se trouvant dans un site de fixation potentiel des facteurs de transcription Th1/E47cs.

Pour la détermination de ce polymorphisme, on prendra comme référence la séquence décrite par Paik et al. (1985, Proc. Natl Aca. Sci., vol. 82, p. 3447).

Ce polymorphisme a été dénommé par les inventeurs Th1/E47cs pour Th1/E47cs consensus, dans la mesure où il se situe dans une séquence consensus de fixation du facteur de transcription Th1/E47cs.

La mutation mise en évidence se caractérise par la substitution d'une Thymine en Guanine (G → T) dans la séquence :

$$\text{GGGTGTCTGT(ou G)ATTACTGGG,}$$

G étant l'allèle le plus fréquent dans la population normale.

Les allèles correspondant à ce polymorphisme sont dénommés ci-après T (lorsque la base est la Thymine) ou G (lorsque la base est la Guanine).

La détermination de l'allèle peut être réalisée après amplification par PCR de la région d'ADN comprenant ce polymorphisme :

- soit en créant dans une des amorces de la PCR un site de clivage pour une enzyme de restriction, n'existant pas chez les individus porteurs d'un des allèles,
- soit par une technique d'hybridation utilisant des sondes oligonucléotidiques spécifiques des allèles.

Les inventeurs ont étudié l'influence du polymorphisme Th1/E47cs sur l'expression des allèles du gène de l'APOE et mis en évidence une augmentation du risque de développer la maladie d'Alzheimer associé à l'allèle T de Th1/E47cs, propre à cet allèle et non dû à l'allèle e4.

De plus, le polymorphisme Th1/E47cs module le risque associé à l'allèle $\varepsilon$4 chez les individus génotypés $\varepsilon$3/$\varepsilon$4, les individus génotypés GT présentant un risque accru de développer la maladie d'Alzheimer par rapport aux individus homozygotes pour le polymorphisme Th1/E47cs. Confortant cette observation, les auteurs ont estimé que l'association de l'allèle T de Th1/E47cs avec l'allèle $\varepsilon$4 sur le même chromosome correspond à la combinaison la plus défavorable.

L'invention a ainsi pour objet un procédé de diagnostic de la maladie d'Alzheimer, comprenant la mise en évidence d'une ou plusieurs mutations dans la région d'ADN génomique de régulation de l'expression du gène de l'apolipoprotéine E, induisant une modification de l'expression du gène de l'apolipoprotéine E par rapport à une population témoin ou une modification de l'expression relative des allèles du gène de l'apolipoprotéine E.

Par diagnostic, au sens de la présente invention, on entend la confirmation d'une mutation dans la région de régulation du gène APOE chez un patient dont le tableau clinique fait état d'une symptomatologie pouvant être attribuée à la maladie d'Alzheimer, ou encore une probabilité accrue chez des sujets de développer la maladie d'Alzheimer par rapport à l'ensemble d'une population, l'accroissement de la probabilité étant statistiquement significative.

La région d'ADN chromosomique de régulation du gène APOE est définie de manière large comme étant la région chromosomique 19q13.2 autre que la région codant pour l'apolipoprotéine E (Human Molecular Genetics, 1994, vol. 3, n° 4, 569-574).

Avantageusement, la région d'ADN chromosomique dans laquelle on met en évidence une ou plusieurs mutations est située entre le marqueur D19S178 et le gène APOCII, et comprend plus particulièrement les introns et les régions flanquantes du gène APOE, s'étendant sur une distance de 5kb en amont et en aval du gène APOE.

L'invention a plus particulièrement pour objet un procédé de diagnostic de la maladie d'Alzheimer comprenant la mise en évidence d'au moins une mutation dans le promoteur du gène APOE, située à 186 bases de la boîte TATA de ce gène, la mutation consistant plus particulièrement en le remplacement T $\rightarrow$ G dans la séquence définie ci-dessus.

Plus particulièrement, le procédé consiste à rechercher une ou plusieurs mutations dans la région du promoteur du gène codant pour l'apolipoprotéine E notamment se trouvant dans un site de fixation potentiel des facteurs de transcription Th1/E47.

L'invention a également pour objet une méthode de diagnostic de la maladie d'Alzheimer comprenant la détermination du génotype de l'apolipoprotéine E et la recherche d'une mutation du type décrit ci-dessus dans la région de régulation du gène APOE.

Conformément à cette méthode de diagnostic, la présence d'au moins un allèle $\varepsilon$4 de l'apolipoprotéine E conjointement à l'existence d'une mutation dans la région de régulation du gène APOE, en particulier la mutation définie ci-dessus induisant une modification de l'expression du gène APOE ou une différence d'expression relative des allèles de l'apolipoprotéine E le cas échéant, orientera vers le diagnostic d'une maladie d'Alzheimer chez des patients dont le tableau clinique présente une symptomatologie pouvant être attribuée à une maladie d'Alzheimer ou permettra de classer ses sujets en bonne santé dans une catégorie à risque accru de développer une maladie d'Alzheimer.

Par différence d'expression relative des allèles, on entend une différence d'expression d'une allèle par rapport à l'autre, indépendamment du niveau absolu d'expression du gène.

La recherche de l'allèle $\varepsilon$4 est faite par toute méthode appropriée basée sur la présence d'un résidu ARG en position 112 de l'apolipoprotéine E pour l'allèle $\varepsilon$4, d'un résidu CYS en position 158 pour l'allèle $\varepsilon$2, par rapport aux résidus CYS et ARG dans ces positions pour l'isoforme $\varepsilon$3, la plus répandue.

La mise en évidence d'une mutation supplémentaire dans les régions de régulation du gène APOE telles que définies ci-dessus est réalisée par toute méthode appropriée, notamment un procédé de diagnostic de la maladie d'Alzheimer comprenant la mise en évidence d'au moins une mutation dans le promoteur du gène codant APOE, située à 186 bases de la boîte TATA de ce gène.

La présence d'au moins un allèle $\varepsilon$4 du gène APOE, d'au moins un allèle court du marqueur D19S178 et d'au moins un allèle long du gène APO CII comme décrit dans FR-2 716 894 et l'existence d'au moins une mutation dans la région de régulation du gène APOE contribueront fortement à orienter le diagnostic de la maladie d'Alzheimer chez des sujets symptomatiques ou constitueront un facteur de risque important chez des sujets asymptomatiques.

Dans la mesure où la (les) mutation(s) impliquée(s) dans la maladie d'Alzheimer sont à l'origine d'une variation de l'expression relative des allèles du gène APOE dans le cerveau, il est également possible de déterminer, au lieu de ou en complément d'une mutation dans les régions de régulation du gène APOE définies ci-dessus, le taux d'expression du gène APOE et de comparer ce taux d'expression à celui de la population générale.

La méthode de diagnostic basée sur la détermination du taux d'expression de l'apolipoprotéine E est particulièrement intéressante chez les sujets hétérozygotes, notamment porteurs de l'allèle e4. Chez ces sujets, la méthode de diagnostic au sens de l'invention comprend avantageusement la détermination du taux d'expression de l'allèle $\varepsilon$4

par rapport à l'allèle ε2 ou ε3.

De la même manière, la méthode de diagnostic chez les individus génotypés ε2/ε3 comprend avantageusement la détermination du taux d'expression de l'allèle ε2 par rapport à l'allèle ε3.

Une augmentation ou une diminution significative, respectivement, du taux d'expression/de transcription de l'allèle ε4 chez un sujet hétérozygote ε4ε2 ou ε4ε3 et de l'allèle ε2 chez un sujet hétérozygote ε2ε3, orientera le diagnostic vers une démence de type Alzheimer, si par ailleurs le sujet présente un tableau clinique évoquant la symptomatologie de la maladie d'Alzheimer. Chez un sujet ne présentant pas de signes cliniques apparents, l'augmentation ou la diminution d'expression des allèles ε4 et ε2 respectivement, sera une indication d'une probabilité accrue chez ce sujet de développer ultérieurement la maladie d'Alzheimer.

La détermination du taux d'expression du gène APOE et plus particulièrement des allèles ε4 et ε2 du gène de l'APOE est réalisé avantageusement par mesure du taux relatif des ARNm du gène APOE soit en établissant le rapport de transcription de l'allèle ε4 part rapport à l'allèle ε2 ou ε3 dans le cas des individus génotypés ε4ε2 et ε4ε3, soit en établissant le rapport de transcription de l'allèle ε2 par rapport à l'allèle ε3 dans le cas des individus génotypés ε2ε3.

La mesure du taux de transcription est réalisée suite à l'extraction d'ARNm de tissus biopsiques ou de cellules en cultures et amplification par RT-PCR (Réaction de Polymérisation en chaîne par transcription inverse), à l'aide d'amorces appropriées spécifiques de l'allèle dont on cherche à mesurer le taux d'expression.

Le tissu utilisé est par exemple issu d'une biopsie de tissu cérébral, notamment de lobes frontaux, permettant ainsi la mesure du taux d'expression des allèles de l'APOE dans le cerveau. Il est également possible de déterminer le taux de transcription des ARNm de l'APOE dans des lymphocytes ou des fibroblastes mis en culture cellulaire. De façon générale il sera possible de déterminer le taux de transcription des allèles de l'APOE dans n'importe quels tissus susceptible de présenter une variation du pourcentage d'expression d'un allèle par rapport à un autre entre les patients et les malades. De même cette méthode peut s'appliquer également pour la mise au point et l'usage de modèles cellulaires ou animaux utilisant les allèles de l'APOE.

Le rapport de l'expression des allèles du gène APOE est déterminé de la manière suivante :

a) on soumet l'ADNc à une amplification par PCR en présence d'amorces permettant l'amplification spécifique d'au moins une séquence polymorphe des allèles ;

b) on soumet l'ADN amplifié à l'action d'au moins une enzyme de restriction, permettant la différenciation des allèles ;

c) on sépare les fragments d'ADN ;

d) on évalue la quantité de fragments obtenus au moyen d'un marqueur émettant un signal détectable ;

e) on détermine le rapport initial en les différents allèles, par la formule suivante :

$$\frac{N_{0\ \text{allèle 1}}}{N_{o\ \text{allèle 1}} + N_{o\ \text{allèle 2}}} = \frac{A\alpha'_{\text{allèle 1}}}{A\alpha'_{\text{allèle 1}} + \alpha'_{\text{allèle 2}}}$$

dans laquelle $N_o$ est le nombre initial de molécules d'ADN,

A est le coefficient de proportionnalité permettant de corriger la différence de taille entre les différents fragments de restriction et est égal au rapport des longueurs de fragments de restriction caractéristiques de chaque allèle, et $\alpha'$ est déterminé

• soit par la formule suivante :

$$\alpha' = \frac{DO_{max}}{K'}$$

dans laquelle $DO_{max}$ est la densité optique maximale qui peut être mesurée,

K' est une constante,

$DO_{max}$ et K' étant déterminés par la fonction f suivante :

$$DO = f(V) = \frac{DO_{max}}{(K'+V)} \cdot V$$

dans laquelle V est le volume de l'échantillon obtenu par PCR soumis à l'étape c) et DO est la densité optique mesurée ;

- soit par la fonction g suivante :

$$\frac{1}{DO} = g\left(\frac{1}{V}\right) = \frac{1}{\alpha'} \times \frac{1}{V} + \frac{1}{DO_{max}}$$

dans laquelle DO est la densité optique mesurée, V est le volume de l'échantillon obtenu par PCR soumis à l'étape c) et $DO_{max}$ est la densité optique maximale qui peut être mesurée ;

le coefficient d'amplification $E_1$ de l'ADN contenant l'allèle 1 étant identique au coefficient d'amplification $E_2$ de l'ADN contenant l'allèle 2, pour les différents allèles de l'APOE.

L'ADN amplifié dans l'étape a) est selon l'invention un ADNc comprenant les séquences alléliques d'intérêt, obtenu à partir d'ARNm par la technique usuelle de RT-PCR. Les allèles sont différenciés selon les étapes b) et c) décrites ci-dessus, qui mettent en évidence les polymorphismes de restriction (RFLP).

La séparation des fragments d'ADN selon l'étape c) peut être réalisée notamment par électrophorèse sur gel, préférentiellement par électrophorèse sur gel de polyacrylamide.

Dans le cas du gène APOE, les allèles $\varepsilon 3$, $\varepsilon 2$ et $\varepsilon 4$ peuvent être caractérisés par des fragments de restriction respectivement de 91 pb, 83 pb et 72 pb.

Le coefficient de proportionnalité A peut donc être calculé comme étant A= 91/72 pour les allèles $\varepsilon 3/\varepsilon 4$, A étant 83/72 pour les individus $\varepsilon 2/\varepsilon 4$ et A étant 91/83 pour les individus $\varepsilon 2/\varepsilon 3$. Pour ce dernier génotype, puisque les deux allèles $\varepsilon 2$ et $\varepsilon 3$ donnent une longueur de fragment de restriction de 91 pb, on a la relation $ADO_{allèle\ 1} + DO_{allèle\ 2} = DO_{91\ pb}$.

Cette méthode est en outre particulièrement simple. Il suffit, en effet, soit de reporter la valeur de la densité optique (DO) en fonction du volume V de l'échantillon, la courbe représentative de la fonction f telle que DO = f(V) pouvant être tracée en utilisant la méthode des moindres carrés, et de déterminer $DO_{max}$ et K' à partir de cette courbe ; soit de reporter la valeur 1/Do en fonction de l'inverse du volume de l'échantillon, à savoir 1/V, la courbe représentative de la fonction g telle que

$$\frac{1}{DO} = g\left(\frac{1}{V}\right)$$

pouvant être tracée par régression linéaire, la pente de la droite obtenue étant égale à l'inverse de $\alpha'$.

L'invention a également pour objet un procédé de diagnostic de la maladie d'Alzheimer comprenant les déterminations de la phase des différents polymorphismes e, etTh1/E47cs et éventuellement de -491 AT et 1E1 susceptibles d'entraîner un risque accru de développer la maladie d'Alzheimer.

Au niveau de la population génotypée $\varepsilon 3\varepsilon 4$, le risque associé au génotype GT est supérieur à celui associé au génotype GG. Cette observation pourrait être expliquée en tenant compte de la phase des polymorphismes Th1/E47cs et $\varepsilon 2$, $\varepsilon$ ou $\varepsilon 4$ de l'APOE. En effet, au niveau des hétérozygotes GT et $\varepsilon 3\varepsilon 4$, le risque de développer la maladie dépend de l'association de l'allèle G et de l'allèle $\varepsilon 4$ sur le même chromosome, déterminant ainsi la phase des polymorphismes $\varepsilon$ et Th1/E47cs de l'APOE. Suivant cette phase, deux possibilités existent : (i) un taux d'expression plus important de $\varepsilon 4$, (ii) un taux d'expression plus important de l'allèle $\varepsilon 3$. La première possibilité entraînerait une réponse physiologique en faveur des propriétés de l'isomorphe APO$\varepsilon 4$ et expliquerait un effet plus marqué au niveau des individus porteurs de génotypes $\varepsilon 3\varepsilon 4$ et GT. Cette différence du taux d'expression liée à la phase sera vraie pour tous génotypes hétérozygotes. Cependant, le risque associé à l'allèle G est probablement atténué en raison des différentes combinaisons possibles entre les polymorphismes $\varepsilon$ et Th1/E47cs de l'APOE.

Conformément à l'invention la phase des polymorphismes de l'APOE et de Th1/E47cs est étudiée de la manière suivante :

Un fragment de 4600 pb contenant les polymorphismes ε de l'APOE et Th1/E47cs est amplifié par PCR. Cette amplification est réalisée en utilisant le kit : extend long template PCR system (Boehringer) avec pour oligonucléotide sens :

$$5'\text{-}GGGGGAGGTGCTGGAATCT\text{-}3'$$

et pour oligonucléotide antisens :

$$5'\text{-}CAGATGCGTGAAACTTGGTGA\text{-}3'.$$

Le produit d'amplification est ensuite digéré par l'enzyme de restriction *Afl III*, dont le seul site de coupure sur le fragment amplifié permet de différencier l'allèle ε4 de l'allèle ε3. Après migration du produit de digestion sur gel d'agarose à 0,8 %, l'ADN est transféré sur membrane de nitrocellulose et fixé sous UV. La discrimination entre l'allèle T et l'allèle G est réalisée par un protocole similaire à celui utilisé pour le génotypage par ASO de ce polymorphisme.

En fonction des phases des polymorphismes APOE et Th1/E47cs, il sera possible de définir des sous-groupes de sujets malades et de déterminer pour chaque sous-groupe la thérapie optimale. De même, la détermination de la phase avec d'autres polymorphismes des régions régulatrices du gène de l'APOE susceptibles d'influencer (ou non) son expression, tels que -491 AT décrit par Bullido et al., 1E1, APO CI, APO CII et D19S178 pourrait également s'avérer utile pour la détermination de sous groupes de sujets à risque élevé de développer la maladie.

Ainsi, on sait que les sujets atteints de la maladie d'Alzheimer ont une prédisposition à répondre à certaines thérapies, notamment aux thérapies de type cholinomimétique en fonction du type et du nombre de copies des allèles du gène de l'APOE.

L'invention a donc également pour objet une méthode d'identification de sujets atteints de la maladie d'Alzheimer susceptibles de répondre à une thérapie donnée, par exemple de type cholinomimétique comprenant :

a) la détermination du génotype de l'apolipoprotéine E ;
b) la détermination du génotype du polymorphisme Th1/E47cs; et
c) éventuellement, la détermination de la phase des polymorphismes de l'apolipoprotéine E et de Th1/E47cs.

Les différents polymorphismes de l'APOE et de Th1/E47cs peuvent être mis à profit pour créer des modèles animaux ou cellulaires exprimant mieux ou moins bien l'APOE, utilisés seuls ou en combinaison avec les facteurs génétiques susceptibles d'influencer le développement de la pathologie tels que l'APP, PS1, PS2, etc.. ou les autres marqueurs de la maladie d'Alzheimer comme la phosphorylation anormale de la protéine Tau.

L'invention a donc également pour objet un vecteur de transfection de cellules eucaryotes comprenant au moins un allèle du gène APOE et un allèle du polymorphisme Th1/E47cs et éventuellement un ou plusieurs autres allèles d'autres gènes ou marqueurs proches de ce gène, susceptibles de modifier le risque de développer une maladie d'Alzheimer par rapport à une population normale.

Ces vecteurs peuvent être utilisés pour la production de cellules eucaryotes transfectées ou d'animaux transgéniques.

Un autre objet de l'invention est donc constitué par les cellules eucaryotes transfectées au moyen d'un vecteur tel que défini ci-dessus ou des animaux transgéniques produits au moyen d'un tel vecteur.

On donnera ci-après les résultats d'une étude des taux d'expression des ARNm du gène APOE dans le cerveau.

## EXEMPLE 1:

**Expression dans le cerveau du gène APOE chez des malades atteints de la maladie d'Alzheimer par rapport à des témoins sains**

1) Extraction de l'ARN et amplification des transcripts

Les tissus ont été choisis chez des témoins âgés de 65 ans ou plus et chez des patients atteints de la maladie d'Alzheimer à survenue tardive, dont le diagnostic a été confirmé par examen neuropathologique, avec des génotypes APOE hétérozygotes.

L'extraction de l'ARN a été réalisée sur des échantillons de lobes frontaux telle que décrit dans J.Biol.Chem. 247, 4621-4627 (1972), ou en utilisant un kit d'extraction (QIAGEN) puis l'ARN a été digéré par DNase (Eurogentec). La RT-PCR a été effectuée à l'aide des amorces décrites dans J. Lipid. res. 31, 545-548 (1990). La réaction de transcription inverse a été conduite pendant 1 h 30 à 37° C, à l'aide de l'amorce F4 (5'-ACAGAATTCGCCCCGGCCTGGTA-3') à 50 pmolaire avec 1 μg d'ARN total comme matrice pour la transcriptase inverse M-MLV, conformément aux instructions du fabricant (Gibco/BRL). La PCR a été effectuée à 94° C pendant 10 minutes, suivie de 30 cycles à 58° C pendant 1 minute, 72° C pendant 1 minutes et 94° C pendant 1 minute.

Le volume réactionnel de la PCR de 25 μl contenait l'amorce F6 (5'-TAA GCT TGC CAC GGC TGT CCA AGG A-3') à 50 pmolaire, les dNTP à 0,5 mM, du MgCl$_2$ à 0,1 mM, du triton X-100 à 0,1%, du glycérol à 15 % et de la Taq-Polymérase à 0,05 unités (Eurogentec).

La méthode de calcul de l'expression différentielle des ARNm est celle décrite ci-dessus.

La DO a été estimée par le logiciel ®Imagemaster (Pharmacia).

La longueur des fragments de restriction a été déterminée à 91 pb pour l'allèle ε3, 83 pb pour l'allèle ε2 et 72 pb pour l'allèle ε4.

La valeur du coefficient A correspond pour les génotypes ε3ε4 et ε2ε4 à A = 91/72 et A = 83/72 respectivement. La valeur du coefficient A correspond pour les génotypes ε2ε3 à A=91/83. Le génotype ε2ε3 est caractérisé par les bandes à 94 et 83 pb. Dans ce cas, étant donné que les deux allèles ε2 et ε3 donnent des fragments de restriction de 91 pb, AODε2 = ODε3 = OD 91pb.

2) Résultats

Les résultats sont rapportés aux figures 1 à 4 :

- la figure 1 représentant les produits d'amplification par RT-PCR après coloration;
- la figure 2 représentant l'expression des ARNm d'individus hétérozygotes ε2ε3, ε3ε4 et ε2ε4 atteints de la maladie d'Alzheimer et de témoins. Les traits noirs représentent les moyennes. Les flèches représentent les valeurs attendues du taux d'expression de l'allèle ε4 chez les individus génotypes ε2/ε4. Ces valeurs ont été estimées à partir des pourcentages observés pour les allèles ε2 et ε4 chez les individus génotypes ε2/ε4 et ε3/ε4, respectivement.
- la figure 4 représente la mesure du taux d'expression de l'allèle ε4 chez des sujets sains jeunes ne présentant pas de troubles cognitifs au moment du prélèvement et chez des sujets déments présentant un diagnostic probable de maladie d'Alzheimer.

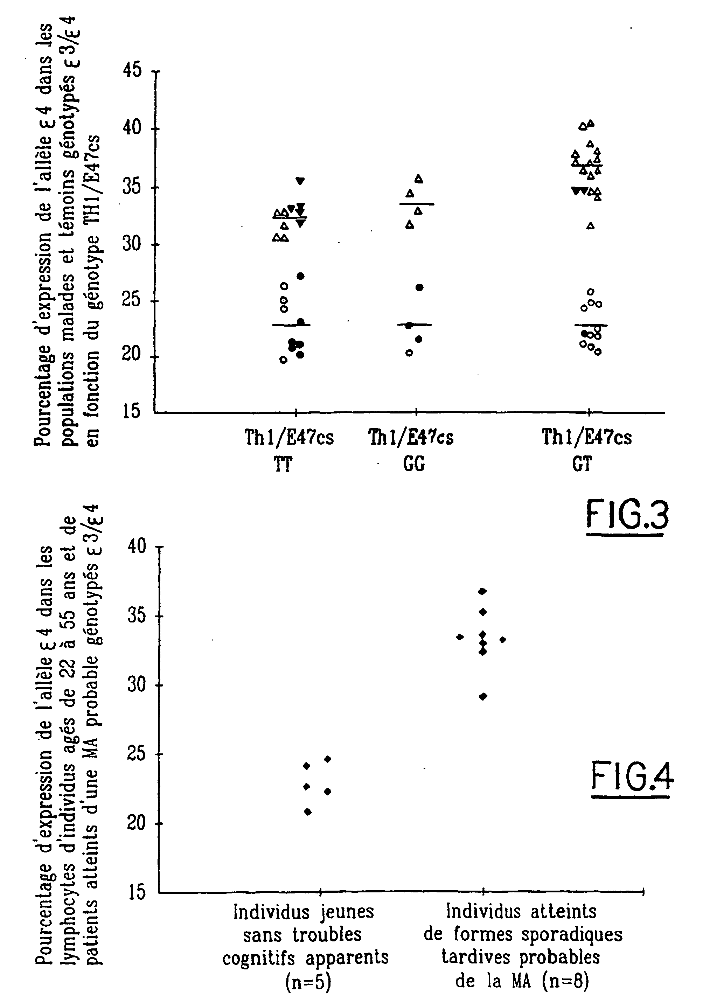

- la figure 3 représente le pourcentage d'expression de l'allèle ε4 chez les patients et témoins en fonction des génotypes Th1/E47cs et -491 AT. Les individus témoins sont représentés par les cercles et les malades par les triangles. Les motifs noirs correspondent aux individus génotypés hétérozygotes pour le polymorphisme -491 AT. Les moyennes d'expression sont représentées par les barres noires.

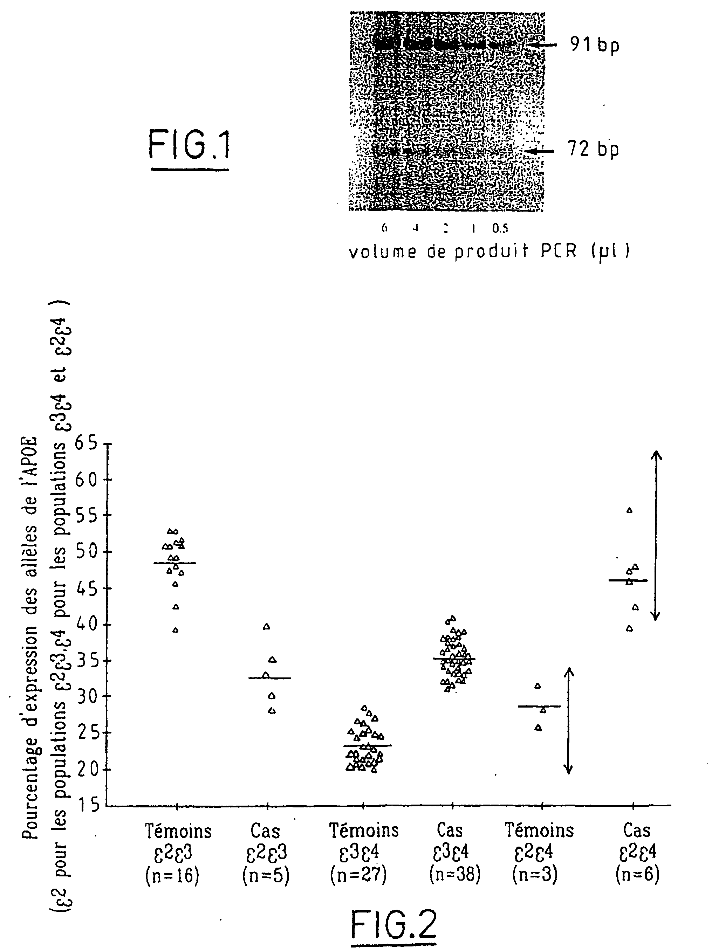

Le nombre de sujets était de 49 pour les patients atteints de la maladie d'Alzheimer et 45 pour les témoins. Les résultats montrent que l'expression de l'ARNm de l'allèle ε3 était dans tous les cas supérieure à celle de l'ARNm de l'allèle ε4, ceci dans tous les cas de figure. Ce résultat est concordant avec les mesures connues des niveaux des protéines APOE trouvés dans les cerveaux des individus dont le génotype a été déterminé. Dans cette étude, il a été montré que le niveau de l'APOE chez les homozygotes ε3 était supérieur à celui des hétérozygotes ε3ε4 qui lui-même était supérieur à celui des homozygotes ε4. Ajoutés les uns aux autres, ces résultats peuvent suggérer soit qu'il existe une différence de stabilité des différentes espèces d'ARNm ou qu'il existe une variabilité génétique de l'expression des deux allèles en déséquilibre avec le polymorphisme génétique. Toutefois; étant donné qu'il existe de plus une différence claire et consistante dans le rapport d'expression allélique entre les patients et les témoins, la seconde hypothèse est probablement la bonne. Les patients atteints de la maladie d'Alzheimer présentent une expression relative plus élevée de l'ARNm ε4 que les témoins ((34,9±2,7 % contre 22,9±2,4 respectivement ; p<0,0001 par un test non paramétrique de Mann et Wihtney) (figure 1). Une augmentation de l'expression de l'allèle ε4 est aussi détectée chez les patients génotypés ε2ε4 comparés aux témoins de même génotype (46,0±5,4% versus 28,0±2,8%, p<0,01). Par contre, une diminution significative de l'expression relative de l'allèle ε2 est mise en évidence dans le cerveau des patients génotypés ε2ε3 par rapport aux témoins de même génotype (32,8±4,5% versus 47,8±3,9%, p<0,002) (figure 1). Confirmant les données obtenues, les valeurs mesurées chez les individus malades ou témoins génotypés e2e4 correspondent aux valeurs estimées à partir des autres génotypes (figure 1).

Dans le but de savoir si la différence d'expression des allèles de l'APOE est spécifique du cerveau ou peut-être retrouvée dans d'autres tissus plus faciles d'obtention du vivant du patient (lymphocytes, fibroblastes... ), les auteurs ont étudié l'expression différentielle des allèles de l'APOE dans les lymphocytes. On donnera ci-après les résultats concernant cette étude.

**EXEMPLE 2 : Expression dans les lymphocytes du gène APOE chez des individus atteints de la maladie d'Alzheimer et des individus jeunes ne présentant pas, au moment de l'étude, de troubles cognitifs.**

1) Séparation des lymphocytes et mise en culture

Un prélèvement sanguin est réalisé sur des individus présentant une maladie d'Alzheimer probable et sur des individus jeunes ne _présentant pas de troubles cognitif au moment de l'étude. Tous ces individus ont la particularité d'être génotypé ε3ε4.

La séparation des lymphocytes est réalisée sur tube Leucosep contenant un gradient de Ficoll (). Après plusieurs lavages des lymphocytes avec du RPMI, ceux-ci sont remis en suspension dans un milieu de culture composé de RPMI, 10% SVF, 2 mM de glutamate, 100 µM de streptopenicilline et 1% de phytohémaaglutinine (Gibco/Brl). La concentration cellulaire est alors de 1 million de cellules par ml. Les cellules en suspension sont mises en culture durant 48 heures par exemple.

2) Extraction de l'ARN et amplification des transcripts

Les méthodes utilisées sont celles décrites précédemment dans l'exemple 1.

3) Résultats

Les auteurs observent un niveau d'expression supérieur de l'allèle ε3 par rapport à l'allèle ε4 chez tous les individus étudiés (qu'ils soient malades ou témoins), recoupant ainsi les observations faites dans le tissu cérébral. Dans les lymphocytes, les malades expriment l'allèle ε4 à un niveau proche de celui observé au niveau des cerveaux des malades génotypés ε3ε4. Sur les 7 individus potentiellement malades, tous présentent un taux d'expression de l'allèle ε4 similaire à celui obtenu à partir d'échantillons cérébraux d'individus pour lesquels le diagnostic de la maladie d'Alzheimer est certain.

Les auteurs ont étudiés l'expression relative de l'allèle ε4 chez 5 individus génotypés ε3ε4 et âgés de 23 à 55 ans ne présentant pas au moment du prélèvement de troubles cognitifs. Ceci a pour but de déterminer si une modification du taux d'expression de l'allèle ε4 peut être visible chez des individus ne présentant aucun signe clinique de la maladie d'Alzheimer. Il pourrait donc exister une possibilité de déterminer de façon précoce les individus à risque plus élevé de développer la maladie d'Alzheimer. Sur les 5 individus étudiés, tous présentent un taux d'expression proche de celui observé dans le cerveau des témoins. Ces résultats suggèrent que la sensibilité et la spécificité de ce test est donc excellente.

En conclusion, il est intéressant de noter que le taux d'expression relatif de l'allèle ε4 serait similaire dans le tissu cérébral de malades de type Alzheimer certains et les lymphocytes de malades Alzheimer de type Alzheimer probables. De même ce taux d'expression serait semblable dans ces deux types cellulaires pour les personnes ne présentant pas de troubles cognitifs. Jusqu'à présent toutes les hypothèses cherchant à impliquer le rôle de l'APOE dans la maladie d'Alzheimer étaient basées sur le fait que les différents allèles de l'APOE étaient exprimés de façon équivalente et à des concentrations identiques dans le cerveau. Les résultats de la présente invention montrent pour la première fois que ce n'est pas le cas et que les allèles sont exprimés de manière différente et que cette différence est significativement plus marquée chez les malades atteints de la maladie d'Alzheimer par rapport à la population témoins. Le même type d'étude pour les autres génotypes hétérozygotes du gène de l'APOE ainsi qu'une quantification absolue du gène de l'APOE quelque soit le génotype est en cours.

En conclusion, les résultats jusqu'à présent obtenus, sont en accord avec l'existence d'une mutation dans les régions régulatrices du gène de l'APOE.

On donnera ci-après les résultats ayant conduit à la mise en évidence du polymorphisme Th1/E47cs.

**EXEMPLE 3 :**

**Caractérisation du polymorphisme Th1/E47cs et impact de celui-ci dans la maladie d'Alzheimer.**

1. Séquençage de l'APOE

Un fragment de 375 paires de bases a été amplifié par PCR avec les amorces pour le brin sens 5'TACTTTCTTTCTGGGATCCAGG 3' et pour le brin antisens 5'ACTCAAGGATCCCAGACTTG 3'. L'amplification est réalisée sur 35 cycles (température d'hybridation des oligonucléotides : 53°C) dans un tampon contenant 1 mM de

MgCl$_2$ final. Le fragment obtenu a été séquencé en suivant les conditions décrites dans les kits de prétraitement (US 70993-Amersham) et séquençage (USB-Amersham-T7 PCR product sequencing kit).

## 2. Détection du polymorphisme poly LBP1 par enzyme de restriction

L'amplification d'un fragment de 228 paires de base contenant le polymorphisme poly LBP1 est réalisée durant 40 cycles en présence de 0,5 mM de MgCl$_2$, 0,5 % en DMSO. La température d'hybridation des oligonucléotides est de 53°C. L'oligonucléotide antisens est identique à celui utilisé pour le séquençage. L'oligonucléotide sens est le suivant : 5'AGAATGGAGGAGGGTGCCTG 3'. En caractère gras, est représenté le nucléotide modifié permettant la création d'un site de coupure avec l'allèle G pour l'enzyme de restriction Bstn *I*. La digestion est effectuée à 60°C toute la nuit. Les produits de digestion sont séparés sur un gel de polyacrylamide à 12% (acrylamide : bisacrylamide 19:1) et colorés dans un bain de bromure d'éthidium. L'allèle T est caractérisé par un fragment de restriction à 49 pb et l'allèle G par un fragment de restriction à 31 pb.

## 3. Détection du polymorphisme par hybridation d'oligonucléotides spécifiques de chaque allèle (Annealing Specific Oligonucleotide ou ASO)

La technique ASO utilisée pour détecter le polymorphisme poly LBP1 est basée sur le protocole décrit par Tiret et al., (1994, Lancet, vol. 344, 910-913). Les modifications spécifiques à la détection du polymorphisme poly LBP1 concernant les oligonucléotides et les températures de lavage sont les suivantes : l'oligonucléotide spécifique de l'allèle G a pour séquence 5'GCCCAGTAATCCAGACACCC 3' avec une température de lavage 61°C, l'oligonucléotide spécifique de l'allèle T a pour séquence : 5' GCCCAGTAATACAGACACCC 3' avec pour température de lavage 62°C.

## 4. Résultats

La première population européenne utilisée dans cette étude comporte 310 témoins (âge=73,5±10,9 ; 37,3% d'hommes) et 293 sujets atteints de formes sporadiques précoces et tardives de la maladie d'Alzheimer (âge=74,6±9,3 ; âge de début de maladie=71,0±8.9; 32,2% d'hommes). Sur cette population ont été testé trois polymorphismes du locus de l'APOE : le polymorphisme Th1/E47cs utilisant les méthodes décrites précédemment. le polymorphisme 1E1 selon la technique utilisée par Mui et collaborateurs (Neurology, 1996, vol. 47, 196-201), le polymorphisme de l'APOE selon la technique de Hixon et Vernier (J. Lipid. Res., vol. 31, 545-548). Le nombre de sujet varie en fonction du polymorphisme étudié : étude de Th1/E47cs, 279 malades et 310 témoins ; étude de 1E1, 293 malades et 307 témoins.

Afin d'améliorer la puissance statistique de l'étude pour les analyses au niveau des individus hétérozygotes pour le génotype de l'APOE, les auteurs ont augmenté le nombre d'individus de génotype ε3/ε4 en ajoutant des sujets de ce génotype à partir de populations indépendantes pour finalement atteindre un total de 152 malades et 91 témoins (respectivement 73,3±8,5 et 70,3±8,5 ans).

La distribution des trois polymorphismes étudiés est présentée dans le tableau 1. Aucune déviation par rapport à l'équilibre de Hardy-Weinberg n'a été observé pour chacun de ces polymorphismes.

La tendance à développer la maladie est exprimée par un facteur d'approximation du risque relatif OR (pour « Odd Ratio »).

IC représente l'intervalle de confiance à 95%.

Les résultats sont rapportés aux tableaux 1 à 5.

Comme attendu, l'allèle ε4 est fortement associé avec la maladie d'Alzheimer (OR=4,67 IC 95% [3,28-6,67]), tandis que l'allèle ε2 montre un effet protecteur (OR=0,27 IC 95% [0,14-0,52]). Dans la population témoin, l'allèle G de Th1/E47cs est le plus représenté (0,531) contrairement à ce qui est observé dans la population malade (0,468). Les distributions allélique et génotypique sont significativement différentes chez les malades par rapport aux témoins (respectivement p=0,03 et p=0,007). L'allèle T de Th1/E47cs est associé à un risque accru de développer la maladie d'Alzheimer (OR=1,29 IC 95% [1,02-1,63]) et l'OR est de 1,79 (IC 95% [1,21-12,65], p=0,002) pour les individus porteurs d'au moins un allèle T. Pour le polymorphisme 1E1, les distributions allélique et génotypique sont significativement différentes entre les deux populations (respectivement p=0,002 et p=0,0005). L'allèle C de 1E1 est associé à une diminution du risque de développer la pathologie (OR=0,56 IC 95% [0,40-0,78], p=0.0003).

Les auteurs ont calculé le déséquilibre de liaison, deux à deux, des différents polymorphismes étudiés (Tableau 2). Un déséquilibre de liaison significatif existe entre les polymorphismes Th1/E47cs, 1E1 et de l'APOE. Puis les auteurs ont estimé les haplotypes générés par ces trois polymorphismes en utilisant l'algorithme Myriad Haplotype (tableau 3). La distribution estimée des haplotypes est significativement différente entre la population des malades et celle des témoins. Par ailleurs, afin de tenir compte de la forte association de l'allèle ε4 avec la maladie d'Alzheimer, les auteurs ont comparé la distribution estimée des haplotypes dans les sous groupes de génotype ε3/ε3 et ε3/ε4, cette distribution étant significativement différente entre les patients et les témoins (tableau 3). Ces résultats suggèrent que les polymorphismes Th1/E47cs et 1E1 ont un effet propre par rapport aux polymorphismes ε de l'APOE.

Dans la population générale (malades=261 et témoins=253), les auteurs ont estimé le risque de développer la maladie d'Alzheimer pour les individus possédant au moins un allèle de chacun des polymorphismes, cette estimation étant réalisée dans un premier temps de façon indépendante entre ces polymorphismes. Un effet significatif est alors observé pour les polymorphismes (Tableau 4). Lorsque tous ces polymorphismes sont pris en compte simultanément dans une régression logistique, les effets de Th1/E47 et 1E1 persistent et augmentent même, appuyant l'hypothèse que plusieurs polymorphismes existeraient à l'intérieur du locus de l'APOE.

Si le niveau d'expression des allèles de l'APOE est augmenté par l'existence de mutations en cis dans la région promotrice du gène de l'APOE, trois implications doivent être vérifiées : (1) la mutation située dans le promoteur doit moduler l'expression des allèles de l'APOE, mettant alors en exergue l'effet délétère de l'allèle ε4 ou bien accentuant l'effet protecteur de l'allèle ε2; (2) étant donné que ce qui détermine le risque serait le niveau relatif d'expression des deux allèles de l'APOE chez les individus hétérozygotes, cette mutation ne doit pas avoir le même impact dans la population de génotype hétérozygote pour l'APOE par rapport à la population de génotype homozygote. (3) l'effet d'une mutation en cis chez les individus hétérozygotes doit dépendre de la phase de ce polymorphisme avec les allèles de l'APOE. (i.e. l'haplotype). Chez les individus porteurs d'une copie de l'allèle ε4 hétérozygote pour le génotype de Th1/E47cs, l'allèle ε4 peut être associé soit avec l'allèle T, soit avec l'allèle G de Th1/E47cs. Une de ces combinaisons devrait augmenter le niveau relatif d'expression de l'allèle ε4 et diminuer celui de l'allèle ε3, l'autre combinaison ayant alors l'effet inverse.

Ainsi chez les individus de génotype ε3/ε4, le risque de développer la maladie d'Alzheimer sera différent entre les individus hétérozygotes et homozygotes pour Th1/E47cs. Afin de tester ces hypothèses, les auteurs ont étudié le risque de développer la pathologie (ajusté sur le sexe et l'âge) des individus hétérozygotes pour Th1/E47cs comparés aux individus homozygotes pour ce même polymorphisme. Seul les individus hétérozygotes pour le génotype Th1/E47cs et pour celui de l'APOE présentent une augmentation de risque de développer la pathologie (OR=2,40 IC 95% [1,40-4,12], p=0,001), cet effet ne se retrouvant pas pour les individus homozygotes pour le génotype de l'APOE (OR=1,02 IC 95% [0,67-1,57], p=0,91 ). Cet impact est mis en évidence en particulier dans la sous-population de génotype ε3/ε4 puisque le risque de développer la maladie d'Alzheimer pour les individus hétérozygotes pour le génotype Th1/E47cs est augmenté par rapport aux individus homozygotes pour ce génotype (OR=1,90 IC 95% [0,97-3,70], p=0,06; malades=107 et témoins=54). Cette augmentation de risque devient significative en élargissant la population de génotype ε3/ε4 (OR=2,07 IC 95% [1,21-3,54], p=0,008; malades=152 et témoins=91) (tableau 5). Par l'utilisation d'une régression logistique pas à pas, les auteurs ont montré que le risque de développer la pathologie est modifié seulement chez les individus hétérozygotes pour le génotype de Th1/E47cs (OR=1,89 IC 95% [1,07-3,35], p=0,027) et pas chez les individus hétérozygotes pour le génotype de 1E1. Ces résultats suggèrent donc qu'entre les deux polymorphismes Th1/E47cs et 1E1, Th1/E47cs serait un meilleur candidat pour modifier le taux relatif d'expression des allèles de l'APOE.

Afin d'appréhender l'influence de la phase des polymorphismes Th1/E47cs et APOE sur le risque de développer la maladie d'Alzheimer, les auteurs ont estimé les fréquences des haplotypes correspondant à l'association des allèles T ou G de Th1/E47cs avec l'allèle ε4 chez les individus de génotype ε3/ε4 que ce soit pour les malades ou les témoins. Pour les individus porteurs des génotypes ε3/ε4 et GT, 57,6% des témoins présenteraient l'association des allèles T et ε4 sur le même chromosome, cette proportion passant à 69,7% chez les malades. L'OR estimé est alors de 1,7 pour les individus présentant cet haplotype, résultat suggérant de nouveau que le polymorphisme Th1/E47cs est susceptible d'influer le taux d'expression de l'allèle ε4.

Afin de confirmer ces résultats, les auteurs ont étudié l'impact du polymorphisme Th1/47cs dans une population plus importante de cas et de témoins. De plus ils ont associé à cette étude un nouveau polymorphisme dans le promoteur du gène de l'APOE, -491 AT (Bullido et al., Nat. Genet, 1998), susceptible lui aussi de modifier le taux d'expression de l'APOE. La population sélectionnée comprend 573 cas sporadiques présentant une maladie d'Alzheimer probable (âge=73,8±8,1 ans, âge de début de maladie=70,4±7,9 ans, 35,9% d'hommes) et 509 témoins (âge=74,9±9,9 ans, 35,9% d'hommes).

Comme précédemment cette population est adaptée pour étudier l'impact du gène de l'APOE dans la maladie d'Alzheimer, puisque l'allèle ε4 est fortement associé avec la pathologie (OR=5,40 IC 95% [4,11-7,09], p<0,0001) tandis que l'allèle ε2 présente un effet protecteur (OR=0,47 IC 95% [0,31-0,70], p=0,0003). La fréquence de l'allèle T du polymorphisme Th1/E47cs est augmentée chez les cas par rapport aux témoins (tableau 6) et le risque de développer la MA pour les individus porteurs d'au moins un allèle T est de 2,13 (IC 95% [1,61-2,83]).

Au niveau de l'étude du polymorphisme -491 AT, tout d'abord les auteurs ont observés une distribution similaire des fréquences alléliques et génotypiques à celles précédemment décrites dans la population nord-américaine. La fréquence de l'allèle T du polymorphisme -491 AT est diminuée chez les patients comparée à celle des témoins et l'OR associé au risque de développer la MA pour les individus porteurs d'au moins un allèle T du polymorphisme -491 AT est de 0,67 (IC 95% [0,52-0,88], p=0,004) (tableau 4).

Afin d'éliminer un biais possible dû à l'allèle ε4 sur l'association des deux polymorphismes Th1/E47cs et -491 AT avec la MA, les auteurs ont testé les effets de chaque allèle en utilisant une régression logistique ajustée sur la présence ou l'absence de l'allèle ε4. Après ajustement, le risque associé à la présence d'au moins d'un allèle T du polymorphisme Th1/E47cs persiste (OR=1,56 IC 95% [1,15-2,11], p=0,004), tandis que le risque associé à la présence d'au moins d'un allèle T du polymorphisme -491 AT disparaît (OR=0,82 IC 95% [0,62-1,10], p=0,19). Cette observation suggère que l'effet de l'allèle T de Th1/E47cs n'est pas expliqué par la présence de l'allèle ε4.Comme précédemment suggéré lors de la première étude, si on assume que le niveau d'expression des allèles de l'APOE est augmenté ou diminué en fonction de mutations cis dans la région du promoteur, ces mutations doivent avoir un effet différent et détectable principalement chez les individus hétérozygotes ε2/ε3/ε4. Afin de vérifier cette hypothèse, les ORs associés au risque de développer la MA ont été étudiés chez les individus hétérozygotes et homozygotes pour le génotype de l'APOE en utilisant une régression logistique ajustée sur l'âge, le sexe, et la présence d'au moins un allèle T des polymorphismes Th1/E47cs et -491 AT. Chez les individus homozygotes ε2/ε3/ε4, aucun effet n'est mis en évidence (OR=1.13 IC 95% [0,77-1,65] et OR=0,99 IC 95% [0,68-1,44] pour les individus porteurs d'au moins un allèle T de Th1/E47cs et de -491 AT, respectivement). Par contre, chez les individus hétérozygotes ε2/ε3/ε4. ceux qui possèdent au moins un allèle T de Th1/E47cs présentent une augmentation de risque de développer la pathologie (OR=3,57 IC 95% [2,22-5,75], p<0,0001), contrairement à ceux porteurs d'au moins d'un allèle T de -491 AT qui présentent un effet protecteur (OR=0,57 IC 95% [0,37-0,90], p=0,014]). Ces observations sont donc en accord avec les hypothèses de départ des auteurs.

Ainsi, les résultats des auteurs. obtenus par deux études épidémiologiques, montrent que la région promotrice du gène de l'APOE possèdent des mutations susceptibles de favoriser le développement de la maladie d'Alzheimer, en particulier le polymorphisme Th1/E47cs, de façon indépendante aux polymorphismes ε du gène de l'APOE. Ce polymorphisme modifie l'impact de l'allèle ε4 dans la population étudiée, définissant au sein de la population ε3/ε4, des sous populations présentant des risques différents, les individus étant hétérozygotes pour le polymorphisme Th1/E47cs présentant le risque le plus important. L'estimation de la phase permet de mieux cerner la sous population la plus à risque, c'est à dire celle présentant l'association de l'allèle ε4 et de l'allèle T sur le même chromosome.

L'influence de ces polymorphismes peut être expliquée par une augmentation du niveau d'expression de l'allèle ε4 ou une diminution de l'expression de l'allèle ε2 par rapport aux autres allèles, cette hypothèse étant confortée par l'analyse de l'expression différentielle des ARNms issus des allèles de l'APOE dans le cerveau des patients et des témoins.

Il sera intéressant d'étudier la phase entre ces deux polymorphismes en utilisant des allèles spécifiques de chacun des polymorphismes.

Ainsi, dans un premier temps, par deux études épidémiologiques, les auteurs ont mis en évidence les effets propres de deux polymorphismes sur le risque de développer la MA, l'allèle T de Th1/E47cs et de -491 AT ayant respectivement un effet délétère et protecteur. Dans un deuxième temps les auteurs ont observé une expression différentielle marquée des allèles de l'APOE, l'allèle ε4 étant surexprimée chez les patients de génotype ε3ε4 et ε2ε4, et l'allèle ε2 étant sous exprimé chez les patients ε2ε3 comparé aux témoins de même génotype. Finalement en accord avec les données déduites des études cas-témoins, le niveau d'expression de l'allèle ε4 dans le cerveau des patients ε3ε4 est corrélé aux mutations présentes dans la région régulatrice du gène de l'APOE. L'allèle T de Th1/E47cs augmente le niveau relatif d'expression de l'allèle ε4 chez les patients, tandis que l'alièle T de -491 AT diminue celui-ci (figure 3 et tableau 5). Ces résultats sont en accord avec des données récentes. suggérant une modification de l'expression du gène de l'APOE par ces deux mutations dans une lignée cellulaire d'hépatome.

Il est intéressant de noter que le polymorphisme Th1/E47cs est localisé dans un site consensus de fixation du facteur de transcription Th1/E47 et plus particulièrement dans le site de fixation du facteur de transcription hélice-boucle-hélice E47. Ce facteur appartient aux protéines de classe A, qui sont exprimées de façon ubiquitaire et forment de multiples combinaisons avec les protéines de classe B pour contrôler l'expression tissu-spécifique des gènes (Murre et collaborateurs, Cell, Vol. 15, 451-459). En particulier, E47 est exprimé dans le cerveau, associé à de multiples protéines de classe B et impliqué dans le développement et la maintenance du système nerveux des mammifères. Par contre peu de choses sont connus du facteur de transcription Th1, identifié lors d'un criblage de banque d'ADNc d'embryon de souris. Une protéine homologue avait été décrite chez la drosophile, associée au facteur de transcription de classe A, Daugtherless, dont l'expression est essentielle dans la neurogénèse.

En conclusion, toutes les données recueillies par les auteurs laissent supposer que l'étude de l'expression du gène de l'APOE permettrait de poser un diagnostic chez les individus hétérozygotes pour le génotype de l'APOE, mais aussi mieux définir des sous-populations à risque. Ces informations seront donc importantes pour définir de nouvelles cibles thérapeutiques et prescrire les traitements optimaux.

Tableau 1: Combinaison des différents allèles des polymorphismes APOE Th1/E47cs et 1E1 dans les populations contrôles et malades.

| APOE | N | Fréquence allélique | | | Fréquence génotypique | | | | | |
|---|---|---|---|---|---|---|---|---|---|---|
| | | ε2 | ε3 | ε4 | ε2/ε2 | ε2/ε3 | ε2/ε4 | ε3/ε3 | ε3/ε4 | ε4/ε4 |
| Témoins | 308 | 0.081 | 0.805 | 0.114 | 0.003 | 0.133 | 0.230 | 0.649 | 0.179 | 0.130 |
| Malades | 292 | 0.034 | 0.639 | 0.327 | - | 0.034 | 0.034 | 0.425 | 0.393 | 0.113 |

| Th1/E47cs | N | $G^a$ | T | | $GG^b$ | GT | TT |
|---|---|---|---|---|---|---|---|
| Témoins | 310 | 0.531 | 0.469 | | 0.320 | 0.422 | 0.258 |
| Malades | 279 | 0.468 | 0.532 | | 0.208 | 0.520 | 0.272 |

| 1E1 | n | $G^c$ | C | | $GG^d$ | GC | CC |
|---|---|---|---|---|---|---|---|
| Témoins | 307 | 0.615 | 0.385 | | 0.388 | 0.456 | 0.156 |
| Malades | 293 | 0.711 | 0.289 | | 0.533 | 0.382 | 0.085 |

**Combinaison des polymorphismes APOE and 1E1**

Malades

| | N | ε2/ε3[*] | ε3/ε3 | ε3/ε4 | ε4/ε4 | ε2/ε4 |
|---|---|---|---|---|---|---|
| CC | 22 | 1 | 20 | - | 1 | - |
| GC | 100 | 4 | 50 | 46 | - | - |
| GG | 139 | 4 | 44 | 56 | 27 | 8 |
| n | 261 | 9 | 114 | 102 | 28 | 8 |

Témoins

| | N | ε2/ε3 | ε3/ε3 | ε3/ε4 | ε4/ε4 | ε2/ε4 |
|---|---|---|---|---|---|---|
| CC | 41 | 0 | 41 | 0 | - | - |
| GC | 112 | 12 | 76 | 24 | - | - |
| GG | 100 | 25 | 50 | 15 | 3 | 7 |
| n | 253 | 37 | 167 | 39 | 3 | 7 |

**Combinaison des polymorphismes APOE and Th1/E47cs**

Malades

| | N | ε2/ε3[*] | ε3/ε3 | ε3/ε4 | ε4/ε4 | ε2/ε4 |
|---|---|---|---|---|---|---|
| GG | 55 | 4 | 36 | 13 | 1 | 1 |
| GT | 134 | 5 | 54 | 60 | 8 | 7 |
| TT | 78 | - | 24 | 29 | 19 | - |
| n | 261 | 9 | 114 | 102 | 28 | 8 |

Témoins

| | N | ε2/ε3 | ε3/ε3 | ε3/ε4 | ε4/ε4 | ε2/ε4 |
|---|---|---|---|---|---|---|
| GG | 85 | 25 | 52 | 5 | - | 3 |
| GT | 108 | 11 | 76 | 16 | 1 | 4 |
| TT | 60 | 1 | 39 | 18 | 2 | - |
| n | 253 | 36 | 167 | 39 | 3 | 7 |

[a] p<0.001: [b] p=0.03. OR(T'/T')=1.29 CI 95% [1.02-1.63]: [c] p=0.007: [d] p=0.002. OR(G'/G')=1.44 CI 95% [1.14-1.84]: [e] p=0.0005: [f] NS. [*] un individu est génotypé ε2/ε2 dans la population témoin.

Tableau 2:

| Déséquilibre de liaison dans la région chromosomique 19q 13.2.region. | | | |
|---|---|---|---|
| | Th1/E47cs | 1E1 | *APOE* |
| **Cas** | | | |
| Th1/E47cs | - | -8.4 | -5.8 |
| 1E1 | 79 | - | -6.1 |
| *APOE* | 49 | 54 | - |
| | | | |
| **Témoins** | | | |
| Th1/E47cs | - | 11.9 | -1.9 |
| 1E1 | 86 | - | -3.7 |
| *APOE* | 32 | 42 | - |

Le polymorphisme APOE a été analyse comme un marqueur biallelique (i.c. allèle 4 versus allèle 2 or 3)

Au dessus de la diagonale du tableau est représenté le coefficient $\Delta$ de déséquilibre de liaison standardisé. En dessous de cette diagonale est représenté le pourcentage D' maximal du déséquilibre de liaison pour les fréquences alléliques données.

Tableau 3: Estimation des haplotypes possibles entre les différents polymorphismes étudiés dans la région chromosomique19q.13.2. Le marqueur anonyme D19S178 a été intégré à cette étude.

**a. Population Générale**

| Haplotype | Polymorphismes | | | | Fréquences estimées des haplotypes | | |
|---|---|---|---|---|---|---|---|
| Nombre | D19S178 | Th1/E47cs | 1E1 | *APOE* | Cas | Témoins | Témoins attendus |
| 1 | S | T | G | ε4⁻ | 0.130 | 0.032 | 0.029 |
| 2 | S | T | C | ε4⁻ | 0.004 | 0.000 | 0.019* |
| 3 | S | G | G | ε4⁻ | 0.031 | 0.006 | 0.035* |
| 4 | L | T | G | ε4⁻ | 0.100 | 0.043 | 0.027 |
| 5 | L | T | C | ε4⁻ | 0.005 | 0.000 | 0.018* |
| 6 | L | G | G | ε4⁻ | 0.044 | 0.022 | 0.033 |
| 7 | L | G | C | ε4⁻ | 0.003 | 0.000 | 0.021* |
| 8 | S | T | G | ε4⁺ | 0.018 | 0.009 | 0.114* |
| 9 | S | T | C | ε4⁺ | 0.127 | 0.183 | 0.072* |
| 10 | S | G | G | ε4⁺ | 0.184 | 0.267 | 0.142* |
| 11 | S | G | C | ε4⁺ | 0.000 | 0.011 | 0.087* |
| 12 | L | T | G | ε4⁺ | 0.020 | 0.014 | 0.113* |
| 13 | L | T | C | ε4⁺ | 0.128 | 0.170 | 0.070* |
| 14 | L | G | G | ε4⁺ | 0.195 | 0.224 | 0.136* |
| 15 | L | G | C | ε4⁺ | 0.011 | 0.019 | 0.084* |
| Nombre de chromosome | | | | | 522 | 506 | |

**b. Population de génotype ε3/ε3**

| Haplotype | Polymorphismes | | | Fréquences estimées des haplotypes | | |
|---|---|---|---|---|---|---|
| nombre | D19S178 | Th1/E47cs | 1E1 | cas | témoins | Témoins attendus |
| 1 | S | T | G | 0.035 | 0.014 | 0.129* |
| 2 | S | T | C | 0.192 | 0.239 | 0.116* |
| 3 | S | G | G | 0.259 | 0.268 | 0.151* |
| 4 | S | G | C | 0.009 | 0.013 | 0.136* |
| 5 | L | T | G | 0.027 | 0.010 | 0.113* |
| 6 | L | T | C | 0.193 | 0.198 | 0.103* |
| 7 | L | G | G | 0.285 | 0.234 | 0.133* |
| 8 | L | G | C | 0.000 | 0.024 | 0.119* |
| Nombre de chromosome | | | | 228 | 334 | |

**c. Population de génotype ε3/ε4**

| Haplotype | Polymorphismes | | | Fréquences estimées des haplotypes | | |
|---|---|---|---|---|---|---|
| Nombre | D19S178 | Th1/E47cs | 1E1 | Cas | Témoins | Témoins attendus |
| 1 | S | T | G | 0.184 | 0.154 | 0.207 |
| 2 | S | T | C | 0.102 | 0.148 | 0.092 |
| 3 | S | G | G | 0.169 | 0.145 | 0.103 |
| 4 | S | G | C | 0.000 | 0.000 | 0.046 |
| 5 | L | T | G | 0.179 | 0.204 | 0.254 |
| 6 | L | T | C | 0.113 | 0.158 | 0.127 |
| 7 | L | G | G | 0.237 | 0.188 | 0.113 |
| 8 | L | G | C | 0.015 | 0.000 | 0.056 |
| Nombre de chromosome | | | | 204 | 78 | |

Tableau 4:

| OR estimés par régression logistique multiple. | | | |
|---|---|---|---|
| OR 95% IC | Th1/E47cs TT+GT/GG | 1E1 CC+GC/GG | *APOE* avec ε4/sans ε4 |
| Non ajusté | 1.79 [1.21-2.65] P=0.002 | 0.56 [0.40-0.78] p=0.0003 | 4.32 [2.98-6.28] p<0.0001 |
| Ajusté sur le sexe et l'âge | 1.90 [1.28-2.83] P=0.002 | 0.58 [0.41-0.82] p=0.002 | 4.66 [3.14-6.93] p<0.0001 |
| Ajusté sur le sexe. l'âge et autres polymorphismes | 2.51 [1.38-4.56] P=0.001 | 0.43 [0.25-0.74] p=0.002 | 3.22 [2.06-5.02] p<0.0001 |

Tableau 5:

| Distributions des génotypes 1E1 et Th1/E47cs dans la population ε3ε4 élargie. | | | | | | |
|---|---|---|---|---|---|---|
| | Polymorphisme 1E1[a] | | | Polymorphisme Th1/E47cs[b] | | |
| | N | GG | GC | GG | GT | TT |
| Témoins | 91 | 50 | 41 | 16 | 38 | 37 |
| Cas | 152 | 67 | 85 | 19 | 89 | 44 |

[a]p=0.10;
[b]p=0.04

Tableau 6. Distribution allélique et génotypique des polymorphismes APOE, Th1/E47cs and -491 AT.

| APOE | Allèle | | | Génotype | | | | | |
|---|---|---|---|---|---|---|---|---|---|
| | ε2 | ε3 | ε4 | ε2ε2* | ε2ε3 | ε2ε4 | ε3ε3 | ε3ε4 | ε4ε4 |
| Témoins | 75(0. | 832(0 | 111(0 | 4(0.0 | 57(0. | 10(0. | 344(0 | 88(0. | 6(0.0 |
| Cas | 40(0. | 699(0 | 407(0 | - | 16(0. | 24(0. | 223(0 | 237(0 | 73(0. |

| Th1/E47cs | G | T | | GG* | GT | TT | | | |
|---|---|---|---|---|---|---|---|---|---|
| Témoins | 562(0 | 456(0 | | 162(0 | 238(0 | 109(0 | | | |
| Cas | 515(0 | 631(0 | | 103(0 | 308(0 | 162(0 | | | |

| -491 AT | A | T | | AA¹ | AT | TT | | | |
|---|---|---|---|---|---|---|---|---|---|
| Témoins | 833(0 | 185(0 | | 343(0 | 147(0 | 19(0. | | | |
| Cas | 993(0 | 153(0 | | 432(0 | 129(0 | 12(0. | | | |

Le nombre d'allèles (fréquence) est présenté.

Les distributions génotypiques sont en équilibre de Hardy-Weinberg dans les populations contrôles.

*$P<10^{-4}$; ¹ $P=0.005$.

Tableau 7.

| Expression relative de l'allèle ε4 chez les patients et les témoins en fonction des polymorphismes Th1/E47cs et -491 AT. | | | | |
|---|---|---|---|---|
| | n | Cas | n | Témoins* |
| **Th1/E47cs** | | | | |
| GG | 4 | 33.5±1.7% | 4 | 22.5±2.5% |
| GT | 19 | 36.3±2.1% | 11 | 22.6±1.9% |
| TT | 10 | 32.4±1.4% | 10 | 22.8±2.7% |

*NS. ᶦ$P<10^{-4}$, ξ$P=0.11$.

Tableau 7.   (suite)

| | n | Cas | n | Témoins* |
|---|---|---|---|---|
| Expression relative de l'allèle ε4 chez les patients et les témoins en fonction des polymorphismes Th1/E47cs et -491 AT. | | | | |
| **Th1/E47cs** | | | | |
| | | | | |
| **-491 AT** | | | | |
| AA | 26 | 35.3±2.7% | 15 | 22.8±2.2% |
| AT | 7 | 33.5±1.3% | 10 | 22.5±2.3% |

*NS. $^{:}P<10^{-4}$, $^{\xi}P=0.11$.

LISTE DE SEQUENCES

(1) INFORMATION GENERALE:

(i) DEPOSANT:

(A) NOM: Institut Pasteur de Lille
(B) RUE: 1 rue du Professeur Calmette
(C) VILLE: Lille
(E) PAYS: France
(F) CODE POSTAL: 59019
(G) TELEPHONE: 0320877912
(H) TELECOPIE: 0320877311

(A) NOM: INSERM
(B) RUE: 101 rue de Tolbiac
(C) VILLE: Paris
(E) PAYS: France
(F) CODE POSTAL: 75013

(ii) TITRE DE L'INVENTION: Procédé de diagnostic de la maladie d'Alzheimer

(iii) NOMBRE DE SEQUENCES: 9

(iv) FORME LISIBLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(v) DONNEES DE LA DEMANDE ACTUELLE: NUMERO DE DEPOT: PCT/FR98/01394

(2) INFORMATION POUR LA SEQ ID NO: 1:

(i) CARACTERISTIQUES DE LA SEQUENCE:

(A) LONGUEUR: 19 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:

    (A) ORGANISME: Homo sapiens

(ix) Caractéristiques:

    (A) nom/clé: caractéristique particulière
    (D) autres informations : fragment du nucléotide 629 au nucléotide 647 de la séquence du variant E4 du gène APOE décrite à la figure 3 de Paik et al, 1985, Proc. Natl. Acad. Sci., vol 82, pp 3445-3449.

(xi) DESCRIPTION DE LA SEQUENCE: SEQ ID NO: 1:

`GGGTGTCTGT ATTACTGGG` 19

(2) INFORMATION POUR LA SEQ ID NO: 2:

    (i) CARACTERISTIQUES DE LA SEQUENCE:

    (A) LONGUEUR: 19 paires de bases
    (B) TYPE: acide nucléique
    (C) NOMBRE DE BRINS: simple
    (D) CONFIGURATION: linéaire

    (ii) TYPE DE MOLECULE: ADNc

    (ix) Caractéristiques:

    (A) nom/clé: caractéristique particulière
    (D) autres informations : amorce

    (xi) DESCRIPTION DE LA SEQUENCE: SEQ ID NO: 2:

`GGGGGAGGTG CTGGAATCT` 19

(2) INFORMATION POUR LA SEQ ID NO: 3:

    (i) CARACTERISTIQUES DE LA SEQUENCE:

    (A) LONGUEUR: 21 paires de bases
    (B) TYPE: acide nucléique
    (C) NOMBRE DE BRINS: simple
    (D) CONFIGURATION: linéaire

    (ii) TYPE DE MOLECULE: ADNc

    (ix) Caractéristiques:

    (A) nom/clé: caractéristique particulière
    (D) autres informations : amorce

    (xi) DESCRIPTION DE LA SEQUENCE: SEQ ID NO: 3:

`CAGATGCGTG AAACTTGGTG A` 21

(2) INFORMATION POUR LA SEQ ID NO: 4:

    (i) CARACTERISTIQUES DE LA SEQUENCE:

        (A) LONGUEUR: 23 paires de bases
        (B) TYPE: acide nucléique
        (C) NOMBRE DE BRINS: simple
        (D) CONFIGURATION: linéaire

    (ii) TYPE DE MOLECULE: ADNc

    (ix) Caractéristiques:

        (A) nom/clé: caractéristique particulière
        (D) autres informations : amorce

    (xi) DESCRIPTION DE LA SEQUENCE: SEQ ID NO: 4:

```
ACAGAATTCG CCCCGGCCTG GTA                                    23
```

    (2) INFORMATION POUR LA SEQ ID NO: 5:

    (i) CARACTERISTIQUES DE LA SEQUENCE:

        (A) LONGUEUR: 22 paires de bases
        (B) TYPE: acide nucléique
        (C) NOMBRE DE BRINS: simple
        (D) CONFIGURATION: linéaire

    (ii) TYPE DE MOLECULE: ADNc

    (ix) Caractéristiques:

        (A) nom/clé: caractéristique particulière
        (D) autres informations : amorce

    (xi) DESCRIPTION DE LA SEQUENCE: SEQ ID NO: 5:

```
TACTTTCTTT CTGGGATCCA GG                                     22
```

    (2) INFORMATION POUR LA SEQ ID NO: 6:

    (i) CARACTERISTIQUES DE LA SEQUENCE:

        (A) LONGUEUR: 20 paires de bases
        (B) TYPE: acide nucléique
        (C) NOMBRE DE BRINS: simple
        (D) CONFIGURATION: linéaire

    (ii) TYPE DE MOLECULE: ADNc

    (ix) Caractéristiques:

        (A) nom/clé: caractéristique particulière
        (D) autres informations : amorce

(xi) DESCRIPTION DE LA SEQUENCE: SEQ ID NO: 6:

ACTCAAGGAT CCCAGACTTG                                    20

(2) INFORMATION POUR LA SEQ ID NO: 7:

    (i) CARACTERISTIQUES DE LA SEQUENCE:

        (A) LONGUEUR: 20 paires de bases
        (B) TYPE: acide nucléique
        (C) NOMBRE DE BRINS: simple
        (D) CONFIGURATION: linéaire

    (ii) TYPE DE MOLECULE: ADNc

    (ix) Caractéristiques:

        (A) nom/clé: caractéristique particulière
        (D) autres informations : amorce

    (xi) DESCRIPTION DE LA SEQUENCE: SEQ ID NO: 7:

AGAATGGAGG AGGGTGCCTG                                    20

(2) INFORMATION POUR LA SEQ ID NO: 8:

    (i) CARACTERISTIQUES DE LA SEQUENCE:

        (A) LONGUEUR: 20 paires de bases
        (B) TYPE: acide nucléique
        (C) NOMBRE DE BRINS: simple
        (D) CONFIGURATION: linéaire

    (ii) TYPE DE MOLECULE: ADNc

    (ix) Caractéristiques:

        (A) nom/clé: caractéristique particulière
        (D) autres informations : amorce

    (xi) DESCRIPTION DE LA SEQUENCE: SEQ ID NO: 8:

GCCCAGTAAT CCAGACACCC                                    20

(2) INFORMATION POUR LA SEQ ID NO: 9:

    (i) CARACTERISTIQUES DE LA SEQUENCE:

        (A) LONGUEUR: 20 paires de bases
        (B) TYPE: acide nucléique
        (C) NOMBRE DE BRINS: simple
        (D) CONFIGURATION: linéaire

    (ii) TYPE DE MOLECULE: ADNc

(ix) Caractéristiques:

(A) nom/clé: caractéristique particulière
(D) autres informations : amorce

(xi) DESCRIPTION DE LA SEQUENCE: SEQ ID NO: 9:

```
GCCCAGTAAT  ACAGACACCC                                        20
```

**Revendications**

1. Procédé de diagnostic de la maladie d'Alzheimer comprenant la mise en évidence d'une mutation dans la région du promoteur du gène codant pour l'apolipoprotéine E, ladite mutation consiste en GGGTGTCTG(G→ T)ATTACTGGG, G étant l'allèle le plus fréquent dans la population normale..

2. Procédé de diagnostic de la maladie d'Alzheimer selon la revendication 1, **caractérisé en ce que** la mutation est mise en évidence :

   - soit en créant dans l'une des amorces d'amplification par PCR de la région polymorphique un site de clivage pour une enzyme de restriction n'existant pas chez les individus porteurs d'un des allèles,
   - soit après amplification par PCR de la région comprenant ce polymorphisme par une technique d'hybridation utilisant des sondes oligonucléotidiques spécifiques des allèles.

3. Procédé de diagnostic de la maladie d'Alzheimer selon l'une quelconque des revendications précédentes, **caractérisé par** les étapes suivantes :

   a) détermination du génotype de l'apolipoprotéine E, incluant une détermination de l'allèle ε4,
   b) mise en évidence d'une mutation dans la région du promoteur du gène de l'apolipoprotéine E, ladite mutation consistant en GGGTGTCTG(G→ T)ATTACTGGG,

   la présence de l'allèle ε4 de l'apolipoprotéine E et de ladite mutation étant indicatrice d'un risque accru de développer la maladie d'Alzheimer.

4. Procédé de diagnostic de la maladie d'Alzheimer selon l'une quelconque des revendications précédentes, **caractérisé en ce que** l'on détermine en outre le taux d'expression des allèles ε2, ε3 et/ou ε4 chez les individus hétérozygotes pour le génotype de l'APOE.

5. Procédé de diagnostic de la maladie d'Alzheimer selon l'une quelconque des revendications précédentes, **caractérisé en ce que** la mise en évidence d'une mutation telle que définie dans la revendication 1, et/ou la mesure du taux d'expression des allèles de l'APOE telle que définie dans la revendication 4 est réalisée à partir d'un échantillon biologique de tissu cérébral prélevé par biopsie, de lymphocytes ou fibroblastes mis en culture.

6. Vecteurs de transfection de cellules eucaryotes comprenant au moins un allèle du gène de l'apolipoprotéine E (APOE) et un allèle d'une mutation telle que définie dans la revendication 1, susceptibles de modifier le risque de développer la maladie d'Alzheimer par rapport à une population normale.

7. Utilisation d'un vecteur selon la revendication 6, pour la production de cellules eucaryotes transfectées ou d'animaux transgéniques non humains.

8. Cellules eucaryotes transfectées au moyen d'un vecteur tel que défini à la revendication 6.

9. Animaux transgéniques non humains produits au moyen d'un vecteur tel que défini à la revendication 6.

**Patentansprüche**

1. Verfahren zur Diagnose von Alzheimer Krankheit, umfassend Nachweisen einer Mutation in der Region des Promotors des Gens, das das Apolipoproteine E codiert, wobei die Mutation besteht aus GGGTGTCTG(G→T)ATTACTGGG, wobei G das häufigste Allel in der normalen Population ist.

2. Verfahren zur Diagnose von Alzheimer Krankheit nach Anspruch 1, **dadurch gekennzeichnet, dass** die Mutation nachgewiesen wird:

   - entweder durch Erzeugen an einer der Amplifikationsstellen durch PCR der polymorphen Region einer Spaltstelle für ein Restriktionsenzym, die nicht bei Individuen vorkommt, die eines der Allele tragen,
   - oder nach Amplifikation durch PCR der Region, die diesen Polymorphismus umfasst, durch eine Hybridisierungstechnik unter Verwendung von für die Allele spezifischen Oligonucleotidsonden.

3. Verfahren zur Diagnose von Alzheimer Krankheit nach einem der vorhergehenden Ansprüche, das durch die folgenden Schritte **gekennzeichnet** ist:

   a) Bestimmung des Genotyps des Apolipoproteins E, einschließlich einer Bestimmung des Allels ε4,
   b) Nachweisen einer Mutation in der Region des Promotors des Gens des Apolipoproteins E, wobei die Mutation besteht aus GGGTGTCTG(G→T)ATTACTGGG,

   wobei die Gegenwart des Allels ε4 des Apolipoproteins E und der Mutation Anzeichen für ein erhöhtes Risiko der Entwicklung von Alzheimer Krankheit sind.

4. Diagnostisches Verfahren für Alzheimer Krankheit nach einem der vorhergehenden Ansprüche, **dadurch gekennzeichnet, dass** ferner der Expressionsgrad der Allele ε2, ε3 und/oder ε4 bei den heterocygoten Individuen für den APOE-Genotyp bestimmt wird.

5. Verfahren zur Diagnose von Alzheimer Krankheit nach einem der vorhergehenden Ansprüche, **dadurch gekennzeichnet, dass** der Nachweis einer Mutation, wie in Anspruch' 1 definiert, und/oder das Messen des Expressionsgrades der Allele des APOE, wie in Anspruch 4 definiert, mit einer biologischen Probe von durch Biopsie entnommenem Hirngewebe, Lymphozyten oder kultivierten Fibroblasten durchgeführt wird.

6. Transfektionsvektor für eukaryotische Zellen, umfassend mindestens 1 Allel des Apolipoprotein E(APOE)-Gens und 1 Allel einer Mutation, wie in Anspruch 1 definiert, die, bezüglich einer normalen Population, einer Modifikation des Risikos der Entwicklung von Alzheimer Krankheit zugänglich sind.

7. Verwendung eines Vektors nach Anspruch 6 zur Produktion von transfizierten eukaryotischen Zellen oder von nicht menschlichen transgenen Tieren.

8. Eukaryotische Zellen, die mit einem Vektor, wie in Anspruch 6 definiert, transfiziert worden sind.

9. Transgene, nicht menschliche Tiere, die mit einem Vektor, wie in Anspruch 6 definiert, produziert worden sind.

**Claims**

1. Method of diagnosing Alzheimer's disease comprising the detection of a mutation in the region of the promoter of the coding gene for apolipoprotein E, said mutation consisting of GGGTGTCTG(G→T)ATTACTGGG, G being the most frequent allele in the normal population.

2. Method of diagnosing Alzheimer's disease according to Claim 1, **characterised in that** the mutation is detected:

   • either by creating a cleavage site for a restriction enzyme, which does not exist in carrier individuals of one of the alleles, in one of the primers of amplification by PCR in the polymorphic region,
   • or after amplification by PCR of the region comprising this polymorphism by a hybridisation technique using specific oligonucleotide probes of the alleles.

3. Method of diagnosing Alzheimer's disease according to any one of the preceding claims, **characterised by** the following steps:

    a) determination of the genotype of apolipoprotein E including determination of allele ε4;
    b) detection of a mutation in the region of the promoter of the gene of apolipoprotein E,

said mutation consisting of GGGTGTCTG(G→T)ATTACTGGG, the presence of allele ε4 of apolipoprotein E and of said mutation being indicative of an increased risk of developing Alzheimer's disease.

4. Method of diagnosing Alzheimer's disease according to any one of the preceding claims, **characterised in that** the expression rate of alleles ε2, ε3 and/or ε4 in heterozygotes for the genotype APOE is determined.

5. Method of diagnosing Alzheimer's disease according to any one of the preceding claims, **characterised in that** the detection of a mutation as defined in Claim 1, and/or the measurement of the expression rate of alleles of APOE as defined in Claim 4 is/are conducted on the basis of a biological sample of brain tissue taken by biopsy, of lymphocytes or fibroblasts in culture.

6. Transfection vectors of eukaryotic cells comprising at least one allele of the gene of apolipoprotein E (APOE) and an allele of a mutation as defined in Claim 1 capable of modifying the risk of developing Alzheimer's disease with respect to a normal population.

7. Use of a vector according to Claim 6 for the production of transfected eukaryotic cells or of non-human transgenic animals.

8. Eukaryotic cells transfected by means of a vector such as that defined in Claim 6.

9. Non-human transgenic animals produced by means of a vector such as that defined in Claim 6.

FIG.1

91 bp

72 bp

6 4 2 1 0.5

volume de produit PCR (µl)

Pourcentage d'expression des allèles de l'APOE
(ε2 pour les populations ε2ε3,ε4 pour les populations ε3ε4 et ε2ε4 )

| Témoins ε2ε3 (n=16) | Cas ε2ε3 (n=5) | Témoins ε3ε4 (n=27) | Cas ε3ε4 (n=38) | Témoins ε2ε4 (n=3) | Cas ε2ε4 (n=6) |

FIG.2

Pourcentage d'expression de l'allèle ε 4 dans les populations malades et témoins génotypés ε 3/ε 4 en fonction du génotype TH1/E47cs

Th1/E47cs TT    Th1/E47cs GG    Th1/E47cs GT

<u>FIG.3</u>

Pourcentage d'expression de l'allèle ε 4 dans les lymphocytes d'individus âgés de 22 à 55 ans et de patients atteints d'une MA probable génotypés ε 3/ε 4

Individus jeunes sans troubles cognitifs apparents (n=5)

Individus atteints de formes sporadiques tardives probables de la MA (n=8)

<u>FIG.4</u>